# EUROPEAN PATENT APPLICATION

(11) **EP 3 848 473 A1**
(43) Date of publication of application: **14.07.2021**
(21) Application number: 18932607.7
(22) Date of filing: 05.09.2018
(51) Int. Cl.: C12Q 1/68, C40B 50/06

(54) **METHOD FOR BUILDING RNA LIBRARY AND KIT**

(71) Applicant: BGI Shenzhen, Shenzhen, Guangdong 518083 (CN)
(72) Inventor: YANG, Xi, Shenzhen, Guangdong 518083 (CN); WANG, Wenjing, Shenzhen, Guangdong 518083 (CN); XING, Yanru, Shenzhen, Guangdong 518083 (CN); XU, Jinjin, Shenzhen, Guangdong 518083 (CN); CHEN, Fang, Shenzhen, Guangdong 518083 (CN); ZHU, Shida, Shenzhen, Guangdong 518083 (CN)
(74) Representative: Inspicos P/S
(86) International application number: PCT/CN2018/104143
(87) International publication number: WO 2020/047769

(57) **Abstract**

Provided are an RNA library construction method and a specialized kit thereof. The method for preparing an RNA library includes the following steps: extracting RNA and performing fragmentation; adding a tail to 3' end; ligating an adaptor to 5' end and hybridizing with a DNA probe mixture; the DNA probe mixture is composed of several DNA probes that are reverse complementary to an RNA that is expected to be removed; removing RNA from the hybrid and removing DNA; performing reverse transcription and PCR amplification to obtain a library solution. The present invention combines poly A tailing and 5'end ligation. The RNA content in the system can be quickly increased by polyA tailing, thereby avoiding subsequent purification losses.

SEQUENCE LISTING

<110>  BGI SHENZHEN

<120>  Method and kit for construction of RNA library

<130>  IEC210036

<160>  208

<170>  PatentIn version 3.5

<210>  1
<211>  29
<212>  RNA
<213>  Artificial sequence

<220>
<221>  misc_feature
<222>  (26)..(29)
<223>  n is a, c, g, or u

<400>  1
gaacgacaug gcuacgaucc gacuunnnn                                          29

<210>  2
<211>  45
<212>  DNA
<213>  Artificial sequence

<220>
<221>  misc_feature
<222>  (45)..(45)
<223>  n is a, c, g, or t

<400>  2
gaccgcttgg cctccgactt tttttttttt tttttttttt tttvn                        45

<210>  3
<211>  59
<212>  DNA
<213>  Artificial sequence

<220>
<221>  misc_feature
<222>  (18)..(27)
<223>  n is a, c, g, or t

<400>  3
tgtgagccaa ggagttgnnn nnnnnnnttg tcttcctaag accgcttggc ctccgactt         59

<210>  4
<211>  25
<212>  DNA
<213>  Artificial sequence

<400>  4

gaacgacatg gctacgatcc gactt                                                25

<210>  5
<211>  50
<212>  DNA
<213>  Artificial sequence

<400>  5
taatgatcct tccgcaggtt cacctacgga aaccttgtta cgacttttac                    50

<210>  6
<211>  50
<212>  DNA
<213>  Artificial sequence

<400>  6
ttcctctaga tagtcaagtt cgaccgtctt ctcagcgctc cgccagggcc                    50

<210>  7
<211>  50
<212>  DNA
<213>  Artificial sequence

<400>  7
gtgggccgac cccggcgggg ccgatccgag ggcctcacta aaccatccaa                     50

<210>  8
<211>  50
<212>  DNA
<213>  Artificial sequence

<400>  8
tcggtagtag cgacgggcgg tgtgtacaaa gggcagggac ttaatcaacg                    50

<210>  9
<211>  50
<212>  DNA
<213>  Artificial sequence

<400>  9
caagcttatg acccgcactt actcgggaat tccctcgttc atggggaata                    50

<210>  10
<211>  50
<212>  DNA
<213>  Artificial sequence

<400>  10
attgcaatcc ccgatcccca tcacgaatgg ggttcaacgg gttacccgcg                    50

<210>  11
<211>  50
<212>  DNA
<213>  Artificial sequence

<400>  11

cctgccggcg tagggtaggc acacgctgag ccagtcagtg tagcgcgcgt                50

<210>  12
<211>  50
<212>  DNA
<213>  Artificial sequence

<400>  12
gcagccccgg acatctaagg gcatcacaga cctgttattg ctcaatctcg                50

<210>  13
<211>  50
<212>  DNA
<213>  Artificial sequence

<400>  13
ggtggctgaa cgccacttgt ccctctaaga agttggggga cgccgaccgc                50

<210>  14
<211>  50
<212>  DNA
<213>  Artificial sequence

<400>  14
tcgggggtcg cgtaactagt tagcatgcca gagtctcgtt cgttatcgga                50

<210>  15
<211>  50
<212>  DNA
<213>  Artificial sequence

<400>  15
attaaccaga caaatcgctc caccaactaa gaacggccat gcaccaccac                50

<210>  16
<211>  50
<212>  DNA
<213>  Artificial sequence

<400>  16
ccacggaatc gagaaagagc tatcaatctg tcaatcctgt ccgtgtccgg                50

<210>  17
<211>  50
<212>  DNA
<213>  Artificial sequence

<400>  17
gccgggtgag gtttcccgtg ttgagtcaaa ttaagccgca ggctccactc                50

<210>  18
<211>  50
<212>  DNA
<213>  Artificial sequence

<400>  18

ctggtggtgc ccttccgtca attcctttaa gtttcagctt tgcaaccata 50

<210> 19
<211> 50
<212> DNA
<213> Artificial sequence

<400> 19
ctccccccgg aacccaaaga ctttggtttc ccggaagctg cccggcgggt 50

<210> 20
<211> 50
<212> DNA
<213> Artificial sequence

<400> 20
catgggaata acgccgccgc atcgccggtc ggcatcgttt atggtcggaa 50

<210> 21
<211> 50
<212> DNA
<213> Artificial sequence

<400> 21
ctacgacggt atctgatcgt cttcgaacct ccgactttcg ttcttgatta 50

<210> 22
<211> 50
<212> DNA
<213> Artificial sequence

<400> 22
atgaaaacat tcttggcaaa tgctttcgct ctggtccgtc ttgcgccggt 50

<210> 23
<211> 50
<212> DNA
<213> Artificial sequence

<400> 23
ccaagaattt cacctctagc ggcgcaatac gaatgccccc ggccgtccct 50

<210> 24
<211> 50
<212> DNA
<213> Artificial sequence

<400> 24
cttaatcatg gcctcagttc cgaaaaccaa caaaatagaa ccgcggtcct 50

<210> 25
<211> 50
<212> DNA
<213> Artificial sequence

<400> 25

attccattat tcctagctgc ggtatccagg cggctcgggc ctgctttgaa                50

<210>  26
<211>  50
<212>  DNA
<213>  Artificial sequence

<400>  26
cactctaatt ttttcaaagt aaacgcttcg ggccccgcgg gacactcagc                50

<210>  27
<211>  50
<212>  DNA
<213>  Artificial sequence

<400>  27
taagagcatc gagggggcgc cgagaggcaa ggggcgggga cgggcggtgg                50

<210>  28
<211>  50
<212>  DNA
<213>  Artificial sequence

<400>  28
ctcgcctcgc ggcggaccgc ccgcccgctc ccaagatcca actacgagct                50

<210>  29
<211>  50
<212>  DNA
<213>  Artificial sequence

<400>  29
ttttaactgc agcaacttta atatacgcta ttggagctgg aattaccgcg                50

<210>  30
<211>  50
<212>  DNA
<213>  Artificial sequence

<400>  30
gctgctggca ccagacttgc cctccaatgg atcctcgtta aaggatttaa                50

<210>  31
<211>  50
<212>  DNA
<213>  Artificial sequence

<400>  31
agtggactca ttccaattac agggcctcga aagagtcctg tattgttatt                50

<210>  32
<211>  50
<212>  DNA
<213>  Artificial sequence

<400>  32

tttcgtcact acctccccgg gtcgggagtg ggtaatttgc gcgcctgctg 50

<210> 33
<211> 50
<212> DNA
<213> Artificial sequence

<400> 33
ccttccttgg atgtggtagc cgtttctcag gctccctctc cggaatcgaa 50

<210> 34
<211> 50
<212> DNA
<213> Artificial sequence

<400> 34
ccctgattcc ccgtcacccg tggtcaccat ggtaggcacg gcgactacca 50

<210> 35
<211> 48
<212> DNA
<213> Artificial sequence

<400> 35
tcgaaagttg atagggcaga cgttcgaatg ggtcgtcgcc gccacggg 48

<210> 36
<211> 50
<212> DNA
<213> Artificial sequence

<400> 36
gcgtgcgatc ggcccgaggt tatctagagt caccaaagcc gccggcgccc 50

<210> 37
<211> 50
<212> DNA
<213> Artificial sequence

<400> 37
gccccccggc cggggccgga gaggggctga ccgggttggt tttgatctga 50

<210> 38
<211> 50
<212> DNA
<213> Artificial sequence

<400> 38
taaatgcacg catccccccc gcgaaggggg tcagcgcccg tcggcatgta 50

<210> 39
<211> 50
<212> DNA
<213> Artificial sequence

<400> 39

ttagctctag aattaccaca gttatccaag taggagagga gcgagcgacc 50

<210> 40
<211> 50
<212> DNA
<213> Artificial sequence

<400> 40
aaaggaacca taactgattt aatgagccat tcgcagtttc actgtaccgg 50

<210> 41
<211> 50
<212> DNA
<213> Artificial sequence

<400> 41
ccgtgcgtac ttagacatgc atggcttaat ctttgagaca agcatatgct 50

<210> 42
<211> 50
<212> DNA
<213> Artificial sequence

<400> 42
tggcttaatc tttgagacaa gcatatgcta ctggcaggat caaccaggta 50

<210> 43
<211> 50
<212> DNA
<213> Artificial sequence

<400> 43
gacaaaccct tgtgtcgagg gctgactttc aatagatcgc agcgagggag 50

<210> 44
<211> 50
<212> DNA
<213> Artificial sequence

<400> 44
ctgctctgct acgtacgaaa ccccgaccca gaagcaggtc gtctacgaat 50

<210> 45
<211> 49
<212> DNA
<213> Artificial sequence

<400> 45
ggtttagcgc caggttcccc acgaacgtgc ggtgcgtgac gggcgaggg 49

<210> 46
<211> 50
<212> DNA
<213> Artificial sequence

<400> 46

gcggccgcct ttccggccgc gccccgtttc ccaggacgaa gggcactccg 50

<210> 47
<211> 50
<212> DNA
<213> Artificial sequence

<400> 47
caccggaccc cggtcccggc gcgcggcggg gcacgcgccc tcccgcggcg 50

<210> 48
<211> 50
<212> DNA
<213> Artificial sequence

<400> 48
gggcgcgtgg aggggrgggc ggcccgccgg cggggacagg cgggggaccg 50

<210> 49
<211> 50
<212> DNA
<213> Artificial sequence

<400> 49
gctatccgag gccaaccgag gctccgcggc gctgccgtat cgttcgcctg 50

<210> 50
<211> 50
<212> DNA
<213> Artificial sequence

<400> 50
ggcgggattc tgacttagag gcgttcagtc ataatcccac agatggtagc 50

<210> 51
<211> 50
<212> DNA
<213> Artificial sequence

<400> 51
ttcgccccat tggctcctca gccaagcaca tacaccaaat gtctgaacct 50

<210> 52
<211> 50
<212> DNA
<213> Artificial sequence

<400> 52
gcggttcctc tcgtactgag caggattacc atggcaacaa cacatcatca 50

<210> 53
<211> 50
<212> DNA
<213> Artificial sequence

<400> 53

gtagggtaaa actaacctgt ctcacgacgg tctaaaccca gctcacgttc 50

<210> 54
<211> 50
<212> DNA
<213> Artificial sequence

<400> 54
cctattagtg ggtgaacaat ccaacgcttg gcgaattctg cttcacaatg 50

<210> 55
<211> 50
<212> DNA
<213> Artificial sequence

<400> 55
ataggaagag ccgacatcga aggatcaaaa agcgacgtcg ctatgaacgc 50

<210> 56
<211> 50
<212> DNA
<213> Artificial sequence

<400> 56
ttggccgcca caagccagtt atccctgtgg taacttttct gacacctcct 50

<210> 57
<211> 50
<212> DNA
<213> Artificial sequence

<400> 57
gcttaaaacc caaaaggtca gaaggatcgt gaggccccgc tttcacggtc 50

<210> 58
<211> 50
<212> DNA
<213> Artificial sequence

<400> 58
tgtattcgta ctgaaaatca agatcaagcg agcttttgcc cttctgctcc 50

<210> 59
<211> 50
<212> DNA
<213> Artificial sequence

<400> 59
acgggaggtt tctgtcctcc ctgagctcgc cttaggacac ctgcgttacc 50

<210> 60
<211> 50
<212> DNA
<213> Artificial sequence

<400> 60

gtttgacagg tgtaccgccc cagtcaaact ccccacctgg cactgtcccc                50

<210>  61
<211>  50
<212>  DNA
<213>  Artificial sequence

<400>  61
ggagcgggtc gcgcccggcc gggcgggcgc ttggcgccag aagcgagagc                50

<210>  62
<211>  50
<212>  DNA
<213>  Artificial sequence

<400>  62
ccctcgggct cgcccccccg cctcaccggg tcagtgaaaa aacgatcaga                50

<210>  63
<211>  50
<212>  DNA
<213>  Artificial sequence

<400>  63
gtagtggtat ttcaccggcg gcccgcaggg ccgcggaccc cgccccgggc                50

<210>  64
<211>  50
<212>  DNA
<213>  Artificial sequence

<400>  64
ccctcgcggg gacaccgggr gggcgccggg ggcctcccac ttattctaca                50

<210>  65
<211>  50
<212>  DNA
<213>  Artificial sequence

<400>  65
cctctcatgt ctcttcaccg tgccagacta gagtcaagct caacagggtc                50

<210>  66
<211>  50
<212>  DNA
<213>  Artificial sequence

<400>  66
ttctttcccc gctgattccg ccaagcccgt tcccttggct gtggtttcgc                50

<210>  67
<211>  50
<212>  DNA
<213>  Artificial sequence

<400>  67

tggatagtag gtagggacag tgggaatctc gttcatccat tcatgcgcgt 50

<210> 68
<211> 50
<212> DNA
<213> Artificial sequence

<400> 68
cactaattag atgacgaggc atttggctac cttaagagag tcatagttac 50

<210> 69
<211> 50
<212> DNA
<213> Artificial sequence

<400> 69
tcccgccgtt tacccgcgct tcattgaatt tcttcacttt gacattcaga 50

<210> 70
<211> 50
<212> DNA
<213> Artificial sequence

<400> 70
gcactgggca gaaatcacat cgcgtcaaca cccgccgcgg gccttcgcga 50

<210> 71
<211> 50
<212> DNA
<213> Artificial sequence

<400> 71
tgctttgttt taattaaaca gtcggattcc cctggtccgc accagttcta 50

<210> 72
<211> 50
<212> DNA
<213> Artificial sequence

<400> 72
agtcggctgc taggcgccgg ccgaggcgag gcgcgcgcgg aaccgcggcc 50

<210> 73
<211> 50
<212> DNA
<213> Artificial sequence

<400> 73
ccgggggcgg acccggcggg rgggaccggc ccgcggcccc tccgccgcct 50

<210> 74
<211> 50
<212> DNA
<213> Artificial sequence

<400> 74

gccgccgccg ccgccgcgcg ccgaggagga ggggggaacg gggggcggac 50

<210> 75
<211> 49
<212> DNA
<213> Artificial sequence

<400> 75
gggccgggrg ggtagggcgg ggggacgaac cgccccgccc cgccgcccg 49

<210> 76
<211> 50
<212> DNA
<213> Artificial sequence

<400> 76
ccgaccgccg ccgcccgacc gctcccgccc ccagcggacg cgcgcgcgac 50

<210> 77
<211> 50
<212> DNA
<213> Artificial sequence

<400> 77
cgagacgtgg ggtgggggtg gggggcgcgc cgcgccgccg ccgggctccc 50

<210> 78
<211> 50
<212> DNA
<213> Artificial sequence

<400> 78
cgggggcggc cgcgacgccc gccgcagctg gggcgatcca cgggaagggc 50

<210> 79
<211> 50
<212> DNA
<213> Artificial sequence

<400> 79
ccggctcgcg tccagagtcc gcgccgccgc cggccccccg ggtccccggg 50

<210> 80
<211> 50
<212> DNA
<213> Artificial sequence

<400> 80
gcccccctcg cggggacctg cccccgccgg ccgccccggc ggccgccgcg 50

<210> 81
<211> 50
<212> DNA
<213> Artificial sequence

<400> 81

cggcccctgc cgccccgacc cttctccccc cgccgcgccc ccacgcggcg 50

<210> 82
<211> 50
<212> DNA
<213> Artificial sequence

<400> 82
ctcccccggg gaggggggag gacggggagc gggggagaga gagagagaga 50

<210> 83
<211> 50
<212> DNA
<213> Artificial sequence

<400> 83
gggcgcgggg tggggaggga gcgagcggcg cgcgcgggtg gggcggggga 50

<210> 84
<211> 50
<212> DNA
<213> Artificial sequence

<400> 84
gggccgcgag gggggtgccc cgggcgtggg grgggcgcgc gcctcgtcca 50

<210> 85
<211> 50
<212> DNA
<213> Artificial sequence

<400> 85
gccgcggcgc gcgcccagcc ccgcttcgcg ccccagcccg accgacccag 50

<210> 86
<211> 50
<212> DNA
<213> Artificial sequence

<400> 86
cccttagagc caatccttat cccgaagtta cggatccggc ttgccgactt 50

<210> 87
<211> 50
<212> DNA
<213> Artificial sequence

<400> 87
cccttaccta cattgttcca acatgccaga ggctgttcac cttggagacc 50

<210> 88
<211> 50
<212> DNA
<213> Artificial sequence

<400> 88

tgctgcggat atgggtacgg cccggcgcga gatttacacc ctctcccccg                50

<210>  89
<211>  50
<212>  DNA
<213>  Artificial sequence

<400>  89
gattttcaag ggccagcgag agctcaccgg acgccgccgg aaccgcgacg                50

<210>  90
<211>  50
<212>  DNA
<213>  Artificial sequence

<400>  90
ctttccaagg cacgggcccc tctctcgggg cgaacccatt ccagggcgcc                50

<210>  91
<211>  50
<212>  DNA
<213>  Artificial sequence

<400>  91
ctgcccttca caaagaaaag agaactctcc ccggggctcc cgccggcttc                50

<210>  92
<211>  50
<212>  DNA
<213>  Artificial sequence

<400>  92
tccgggatcg gtcgcgttac cgcactggac gcctcgcggc gcccatctcc                50

<210>  93
<211>  50
<212>  DNA
<213>  Artificial sequence

<400>  93
gccactccgg attcggggat ctgaacccga ctccctttcg atcggccgag                50

<210>  94
<211>  50
<212>  DNA
<213>  Artificial sequence

<400>  94
ggcaacggag gccatcgccc gtcccttcgg aacggcgctc gcccatctct                50

<210>  95
<211>  50
<212>  DNA
<213>  Artificial sequence

<400>  95

caggaccgac tgacccatgt tcaactgctg ttcacatgga acccttctcc                50

<210>  96
<211>  50
<212>  DNA
<213>  Artificial sequence

<400>  96
acttcggcct tcaaagttct cgtttgaata tttgctacta ccaccaagat                50

<210>  97
<211>  50
<212>  DNA
<213>  Artificial sequence

<400>  97
ctgcacctgc ggcggctcca cccgggcccg cgccctaggc ttcaaggctc                50

<210>  98
<211>  50
<212>  DNA
<213>  Artificial sequence

<400>  98
accgcagcgg ccctcctact cgtcgcggcg tagcgtccgc ggggctccgg                50

<210>  99
<211>  45
<212>  DNA
<213>  Artificial sequence

<400>  99
gggcggggag cggggcgtgg gcgggaggag gggaggaggc gtggg                      45

<210>  100
<211>  50
<212>  DNA
<213>  Artificial sequence

<400>  100
gggcggggga aggaccccac acccccgccg ccgccgccgc cgccgccctc                50

<210>  101
<211>  50
<212>  DNA
<213>  Artificial sequence

<400>  101
cgacgcacac cacacgcgcg cgcgcgcgcg ccgcccccgc cgctcccgtc                50

<210>  102
<211>  50
<212>  DNA
<213>  Artificial sequence

<400>  102

cactctcgac tgccggcgac ggccgggtat gggcccgacg ctccagcgcc 50

<210> 103
<211> 50
<212> DNA
<213> Artificial sequence

<400> 103
atccattttc agggctagtt gattcggcag gtgagttgtt acacactcct 50

<210> 104
<211> 50
<212> DNA
<213> Artificial sequence

<400> 104
tagcggattc cgacttccat ggccaccgtc ctgctgtcta tatcaaccaa 50

<210> 105
<211> 50
<212> DNA
<213> Artificial sequence

<400> 105
caccttttct ggggtctgat gagcgtcggc atcgggcgcc ttaacccggc 50

<210> 106
<211> 50
<212> DNA
<213> Artificial sequence

<400> 106
gttcggttca tcccgcagcg ccagttctgc ttaccaaaag tggcccacta 50

<210> 107
<211> 50
<212> DNA
<213> Artificial sequence

<400> 107
ggcactcgca ttccacgccc ggctccacgc cagcgagccg ggcttcttac 50

<210> 108
<211> 50
<212> DNA
<213> Artificial sequence

<400> 108
ccatttaaag tttgagaata ggttgagatc gtttcggccc caagacctct 50

<210> 109
<211> 50
<212> DNA
<213> Artificial sequence

<400> 109

aatcattcgc tttaccggat aaaactgcgt ggcgggggtg cgtcgggtct 50

<210> 110
<211> 50
<212> DNA
<213> Artificial sequence

<400> 110
gcgagagcgc cagctatcct gagggaaact tcggagggaa ccagctacta 50

<210> 111
<211> 50
<212> DNA
<213> Artificial sequence

<400> 111
gatggttcga ttagtctttc gcccctatac ccaggtcgga cgaccgattt 50

<210> 112
<211> 50
<212> DNA
<213> Artificial sequence

<400> 112
gcacgtcagg accgctacgg acctccacca gagtttcctc tggcttcgcc 50

<210> 113
<211> 50
<212> DNA
<213> Artificial sequence

<400> 113
ctgcccaggc atagttcacc atctttcggg tcctaacacg tgcgctcgtg 50

<210> 114
<211> 50
<212> DNA
<213> Artificial sequence

<400> 114
ctccacctcc ccggcgcggc gggcgagacg ggccggtggt gcgccctcgg 50

<210> 115
<211> 50
<212> DNA
<213> Artificial sequence

<400> 115
cggactggag aggcctcggg atcccacctc ggccggcgag cgcgccggcc 50

<210> 116
<211> 50
<212> DNA
<213> Artificial sequence

<400> 116

ttcaccttca ttgcgccacg gcggctttcg tgcgagcccc cgactcgcgc 50

<210> 117
<211> 50
<212> DNA
<213> Artificial sequence

<400> 117
acgtgttaga ctccttggtc cgtgtttcaa gacgggtcgg gtgggtagcc 50

<210> 118
<211> 49
<212> DNA
<213> Artificial sequence

<400> 118
gacgtcgccg ccgaccccgt gcgctcgctc cgccgtcccc ctcttcggg 49

<210> 119
<211> 50
<212> DNA
<213> Artificial sequence

<400> 119
gacgcgcgcg tggccccgag agaacctccc ccgggcccga cggcgcgacc 50

<210> 120
<211> 50
<212> DNA
<213> Artificial sequence

<400> 120
cgcccggggc gcactgggga cagtccgccc cgccccccga cccgcgcgcg 50

<210> 121
<211> 50
<212> DNA
<213> Artificial sequence

<400> 121
gcaccccccc cgtcgccggg gcgggggcgc ggggaggagg ggtgggagag 50

<210> 122
<211> 50
<212> DNA
<213> Artificial sequence

<400> 122
cggtcgcgcc gtgggagggg tggcccggcc cccccacgag gagacgccgg 50

<210> 123
<211> 50
<212> DNA
<213> Artificial sequence

<400> 123

cgcgcccccg cgggggagac ccccctcgcg ggggattccc cgcgggggtg 50

<210> 124
<211> 50
<212> DNA
<213> Artificial sequence

<400> 124
ggcgccggga ggggggagag cgcggcgacg ggtctcgctc cctcggcccc 50

<210> 125
<211> 50
<212> DNA
<213> Artificial sequence

<400> 125
gggattcggc gagtgctgct gccggggggg ctgtaacact cggggrgggt 50

<210> 126
<211> 50
<212> DNA
<213> Artificial sequence

<400> 126
ttcggtcccg ccgcccccgc cgccgccgcc accgccgccg ccgccgccgc 50

<210> 127
<211> 50
<212> DNA
<213> Artificial sequence

<400> 127
cccgacccgc gcgccctccc gagggaggac gcggggccgg ggggcggaga 50

<210> 128
<211> 50
<212> DNA
<213> Artificial sequence

<400> 128
cgggggagga ggaggacgga cggacggacg gggccccccg agccaccttc 50

<210> 129
<211> 50
<212> DNA
<213> Artificial sequence

<400> 129
cccgccgggc cttcccagcc gtcccggagc cggtcgcggc gcaccgccgc 50

<210> 130
<211> 50
<212> DNA
<213> Artificial sequence

<400> 130

ggtggaaatg cgcccggcgg cggccggtcg ccggtcgggg gacggtcccc 50

<210> 131
<211> 50
<212> DNA
<213> Artificial sequence

<400> 131
cgccgacccc acccccggcc ccgcccgccc acccccgcac ccgccggagc 50

<210> 132
<211> 50
<212> DNA
<213> Artificial sequence

<400> 132
ccgccccctc cggggaggag gaggaggggc ggcgggggaa gggagggcgg 50

<210> 133
<211> 50
<212> DNA
<213> Artificial sequence

<400> 133
gtggaggggt cgggaggaac ggggggcggg aaagatccgc cgggccgccg 50

<210> 134
<211> 50
<212> DNA
<213> Artificial sequence

<400> 134
acacggccgg acccgccgcc gggttgaatc ctccgggcgg actgcgcgga 50

<210> 135
<211> 50
<212> DNA
<213> Artificial sequence

<400> 135
ccccacccgt ttacctctta acggtttcac gccctcttga actctctctt 50

<210> 136
<211> 50
<212> DNA
<213> Artificial sequence

<400> 136
caaagttctt ttcaactttc ccttacggta cttgttgact atcggtctcg 50

<210> 137
<211> 50
<212> DNA
<213> Artificial sequence

<400> 137

tgccggtatt tagccttaga tggagtttac cacccgcttt gggctgcatt 50

<210> 138
<211> 50
<212> DNA
<213> Artificial sequence

<400> 138
cccaagcaac ccgactccgg gaagacccgg gcgcgcgccg gccgctaccg 50

<210> 139
<211> 50
<212> DNA
<213> Artificial sequence

<400> 139
gcctcacacc gtccacgggc tgggcctcga tcagaaggac ttgggccccc 50

<210> 140
<211> 50
<212> DNA
<213> Artificial sequence

<400> 140
cacgagcggc gccggggagc gggtcttccg tacgccacat gtcccgcgcc 50

<210> 141
<211> 50
<212> DNA
<213> Artificial sequence

<400> 141
ccgcggggcg gggattcggc gctgggctct tccctgttca ctcgccgtta 50

<210> 142
<211> 50
<212> DNA
<213> Artificial sequence

<400> 142
ctgagggaat cctggttagt ttcttttcct ccgctgacta atatgcttaa 50

<210> 143
<211> 50
<212> DNA
<213> Artificial sequence

<400> 143
gactaatatg cttaaattca gcgggtcgcc acgtctgatc tgaggtcgcg 50

<210> 144
<211> 50
<212> DNA
<213> Artificial sequence

<400> 144

aaaccctgtt cttgggtggg tgtgggtata atactaagtt gagatgatat 50

<210> 145
<211> 50
<212> DNA
<213> Artificial sequence

<400> 145
catttacggg ggaaggcgct ttgtgaagta ggccttattt ctcttgtcct 50

<210> 146
<211> 50
<212> DNA
<213> Artificial sequence

<220>
<221> misc_feature
<222> (23)..(23)
<223> n is a, c, g, or t

<400> 146
ttcgtacagg gaggaatttg aangtagata gaaaccgacc tggattactc 50

<210> 147
<211> 50
<212> DNA
<213> Artificial sequence

<400> 147
cggtctgaac tcagatcacg taggacttta atcgttgaac aaacgaacct 50

<210> 148
<211> 50
<212> DNA
<213> Artificial sequence

<400> 148
ttaatagcgg ctgcaccatc gggatgtcct gatccaacat cgaggtcgta 50

<210> 149
<211> 50
<212> DNA
<213> Artificial sequence

<400> 149
aaccctattg ttgatatgga ctctagaata ggattgcgct gttatcccta 50

<210> 150
<211> 50
<212> DNA
<213> Artificial sequence

<400> 150
gggtaacttg ttccgttggt caagttattg gatcaattga gtatagtagt 50

<210>  151
<211>  50
<212>  DNA
<213>  Artificial sequence

<400>  151
tcgctttgac tggtgaagtc ttagcatgta ctgctcggag gttgggttct          50


<210>  152
<211>  50
<212>  DNA
<213>  Artificial sequence

<400>  152
gctccgaggt cgccccaacc gaaattttta atgcaggttt ggtagtttag          50


<210>  153
<211>  50
<212>  DNA
<213>  Artificial sequence

<400>  153
gacctgtggg tttgttaggt actgtttgca ttaataaatt aaagctccat          50


<210>  154
<211>  50
<212>  DNA
<213>  Artificial sequence

<400>  154
agggtcttct cgtcttgctg tgttatgccc gcctcttcac gggcaggtca          50


<210>  155
<211>  50
<212>  DNA
<213>  Artificial sequence

<400>  155
atttcactgg ttaaaagtaa gagacagctg aaccctcgtg gagccattca          50


<210>  156
<211>  50
<212>  DNA
<213>  Artificial sequence

<400>  156
tacaggtccc tatttaagga acaagtgatt atgctacctt tgcacggtta          50


<210>  157
<211>  50
<212>  DNA
<213>  Artificial sequence

<400>  157
gggtaccgcg gccgttaaac atgtgtcact gggcaggcgg tgcctctaat          50

<210> 158
<211> 50
<212> DNA
<213> Artificial sequence

<400> 158
actggtgatg ctagaggtga tgtttttggt aaacaggcgg ggtaagattt 50


<210> 159
<211> 50
<212> DNA
<213> Artificial sequence

<400> 159
gccgagttcc ttttactttt tttaaccttt ccttatgagc atgcctgtgt 50


<210> 160
<211> 50
<212> DNA
<213> Artificial sequence

<400> 160
tgggttgaca gtgagggtaa taatgacttg ttggttgatt gtagatattg 50


<210> 161
<211> 50
<212> DNA
<213> Artificial sequence

<400> 161
ggctgttaat tgtcagttca gtgttttaat ctgacgcagg cttatgcgga 50


<210> 162
<211> 50
<212> DNA
<213> Artificial sequence

<400> 162
ggagaatgtt ttcatgttac ttatactaac attagttctt ctatagggtg 50


<210> 163
<211> 50
<212> DNA
<213> Artificial sequence

<400> 163
atagattggt ccaattgggt gtgaggagtt cagttatatg tttgggattt 50


<210> 164
<211> 50
<212> DNA
<213> Artificial sequence

<400> 164
tttaggtagt gggtgttgag cttgaacgct ttcttaattg gtggctgctt 50

<210> 165
<211> 50
<212> DNA
<213> Artificial sequence

<400> 165
ttaggcctac tatgggtgtt aaatttttta ctctctctac aaggtttttt 50


<210> 166
<211> 50
<212> DNA
<213> Artificial sequence

<400> 166
cctagtgtcc aaagagctgt tcctctttgg actaacagtt aaatttacaa 50


<210> 167
<211> 49
<212> DNA
<213> Artificial sequence

<400> 167
gggatttaga gggttctgtg ggcaaattta aagttgaact aagattcta 49


<210> 168
<211> 50
<212> DNA
<213> Artificial sequence

<400> 168
tcttggacaa ccagctatca ccaggctcgg taggtttgtc gcctctacct 50


<210> 169
<211> 50
<212> DNA
<213> Artificial sequence

<400> 169
ataaatcttc ccactatttt gctacataga cgggtgtgct cttttagctg 50


<210> 170
<211> 50
<212> DNA
<213> Artificial sequence

<400> 170
ttcttaggta gctcgtctgg tttcgggggt cttagctttg gctctccttg 50


<210> 171
<211> 50
<212> DNA
<213> Artificial sequence

<400> 171
caaagttatt tctagttaat tcattatgca gaaggtatag gggttagtcc 50

<210> 172
<211> 50
<212> DNA
<213> Artificial sequence

<400> 172
ttgctatatt atgcttggtt ataatttttc atctttccct tgcggtacta 50


<210> 173
<211> 50
<212> DNA
<213> Artificial sequence

<400> 173
tatctattgc gccaggtttc aatttctatc gcctatactt tatttgggta 50


<210> 174
<211> 50
<212> DNA
<213> Artificial sequence

<400> 174
aatggtttgg ctaaggttgt ctggtagtaa ggtggagtgg gtttggggct 50


<210> 175
<211> 50
<212> DNA
<213> Artificial sequence

<400> 175
gttcgtccaa gtgcactttc cagtacactt accatgttac gacttgtctc 50


<210> 176
<211> 50
<212> DNA
<213> Artificial sequence

<400> 176
ctctatataa atgcgtaggg gttttagtta aatgtccttt gaagtatact 50


<210> 177
<211> 50
<212> DNA
<213> Artificial sequence

<400> 177
tgaggagggt gacgggcggt gtgtacgcgc ttcagggccc tgttcaacta 50


<210> 178
<211> 50
<212> DNA
<213> Artificial sequence

<400> 178
agcactctac tcttagttta ctgctaaatc caccttcgac ccttaagttt 50

<210>  179
<211>  50
<212>  DNA
<213>  Artificial sequence

<400>  179
cataagggct atcgtagttt tctggggtag aaaatgtagc ccatttcttg                50


<210>  180
<211>  50
<212>  DNA
<213>  Artificial sequence

<400>  180
ccacctcatg ggctacacct tgacctaacg tctttacgtg ggtacttgcg                50


<210>  181
<211>  50
<212>  DNA
<213>  Artificial sequence

<400>  181
cttactttgt agccttcatc agggtttgct gaagatggcg gtatataggc                50


<210>  182
<211>  50
<212>  DNA
<213>  Artificial sequence

<400>  182
tgagcaagag gtggtgaggt tgatcggggt ttatcgatta cagaacaggc                50


<210>  183
<211>  50
<212>  DNA
<213>  Artificial sequence

<400>  183
tcctctagag ggatatgaag caccgccagg tcctttgagt tttaagctgt                50


<210>  184
<211>  50
<212>  DNA
<213>  Artificial sequence

<400>  184
ggctcgtagt gttctggcga gcagttttgt tgatttaact gttgaggttt                50


<210>  185
<211>  50
<212>  DNA
<213>  Artificial sequence

<400>  185
agggctaagc atagtggggt atctaatccc agtttgggtc ttagctattg                50

<210>  186
<211>  50
<212>  DNA
<213>  Artificial sequence

<400>  186
tgtgttcaga tatgttaaag ccactttcgt agtctatttt gtgtcaactg          50


<210>  187
<211>  50
<212>  DNA
<213>  Artificial sequence

<400>  187
gagtttttta caactcaggt gagttttagc tttattgggg agggggtgat          50


<210>  188
<211>  50
<212>  DNA
<213>  Artificial sequence

<400>  188
ctaaaacact ctttacgccg gcttctattg acttgggtta atcgtgtgac          50


<210>  189
<211>  50
<212>  DNA
<213>  Artificial sequence

<400>  189
cgcggtggct ggcacgaaat tgaccaaccc tggggttagt atagcttagt          50


<210>  190
<211>  48
<212>  DNA
<213>  Artificial sequence

<400>  190
taaactttcg tttattgcta aaggttaatc actgctgttt cccgtggg          48


<210>  191
<211>  50
<212>  DNA
<213>  Artificial sequence

<400>  191
tgtggctagg ctaagcgttt tgagctgcat tgctgcgtgc ttgatgcttg          50


<210>  192
<211>  50
<212>  DNA
<213>  Artificial sequence

<400>  192
ttccttttga tcgtggtgat ttagagggtg aactcactgg aacggggatg          50

<210>  193
<211>  50
<212>  DNA
<213>  Artificial sequence

<400>  193
cttgcatgtg taatcttact aagagctaat agaaaggcta ggaccaaacc                50


<210>  194
<211>  50
<212>  DNA
<213>  Artificial sequence

<400>  194
aaagcctaca gcacccggta ttcccaggcg gtctcccatc caagtactaa                50


<210>  195
<211>  50
<212>  DNA
<213>  Artificial sequence

<400>  195
ccaggcccga ccctgcttag cttccgagat cagacgagat cgggcgcgtt                50


<210>  196
<211>  50
<212>  DNA
<213>  Artificial sequence

<400>  196
ttccgagatc agacgagatc gggcgcgttc agggtggtat ggccgtagac                50


<210>  197
<211>  50
<212>  DNA
<213>  Artificial sequence

<400>  197
aagcgacgct cagacaggcg tagccccggg aggaacccgg ggccgcaagt                50


<210>  198
<211>  50
<212>  DNA
<213>  Artificial sequence

<400>  198
gcgttcgaag tgtcgatgat caatgtgtcc tgcaattcac attaattctc                50


<210>  199
<211>  50
<212>  DNA
<213>  Artificial sequence

<400>  199
gcagctagct gcgttcttca tcgacgcacg agccgagtga tccaccgcta                50

<210>  200
<211>  49
<212>  DNA
<213>  Artificial sequence

<400>  200
gccagtcaaa tttagcagtg gggggttgta taccaacttt agtgacact                49


<210>  201
<211>  49
<212>  DNA
<213>  Artificial sequence

<400>  201
tgacactaat gttaataagt tctgataacc cactaccatc ggaccagcc                49


<210>  202
<211>  49
<212>  DNA
<213>  Artificial sequence

<400>  202
gactagtcaa gtgcagtagt gagaaggggg gaaagagtag aacaaggag                49


<210>  203
<211>  49
<212>  DNA
<213>  Artificial sequence

<400>  203
gaaagagtag aacaaggagt tcgatctgta actgactgtg aacaatcaa                49


<210>  204
<211>  49
<212>  DNA
<213>  Artificial sequence

<400>  204
actgactgtg aacaatcaat tgagataact cactaccttc ggaccagcc                49


<210>  205
<211>  49
<212>  DNA
<213>  Artificial sequence

<400>  205
ggctagtcaa gtgaagcagt gggagtggag aaggaacaaa gaaatctgt                49


<210>  206
<211>  49
<212>  DNA
<213>  Artificial sequence

<400>  206
aactggttgt gatcaattag ttgtaaacac cactgcactc ggaccagcc                49

<210>  207
<211>  49
<212>  DNA
<213>  Artificial sequence

<400>  207
cagcaagcta gtcaagcgcg gttgtggggg gagacaatgt taaatcaac                    49


<210>  208
<211>  49
<212>  DNA
<213>  Artificial sequence

<400>  208
agacaatgtt aaatcaactt aacaataacc cacaacactc ggaccaact                    49

## Description

### Technical Field

The present application relates to a method and a kit for construction of an RNA library.

### Background Art

Non-invasive testing, including NIPT (non-invasive prenatal testing), is not only a very classic technology, but also an emerging field. Plasma or serum enzymatic testing and NIPT analyze plasma/serum at levels of protein and DNA respectively so as to predict physical health or pregnancy. However, the application of plasma/serum RNA has just started, mainly because RNA is not unstable and plasma/serum RNA is mostly small fragment and its concentration is very low, and thus there is not a very well strategy for construction of library for such low-quality and low-concentration RNA. The current technologies for construction of RNA library are directed to only long RNA or short RNA, and there is not a suitable technology for construction of a library for simultaneous long RNA and short RNA, so that simultaneous study for both long RNA and short RNA requires construction of libraries for them separately.

RNaseH method is a classic method for construction of a library for long RNA. The basic steps comprise: annealing the DNA oligo that is reverse complementary to rRNA with the sample, removing rRNA with RNaseH, and then removing DNA oligo with DNase; reverse transcription (first strand cDNA synthesis), second strand synthesis; gap filling, addition of dA, adaptor ligation, PCR. There are the following problems: the process is very cumbersome, slow, and difficult to the implement; the initial amount of RNA is required to be high; only long fragments of RNA can be detected.

Small RNA library construction comprises the following basic steps: ligating 3' Adaptor, then ligating 5' Adaptor, reverse transcription, PCR. There are the following problems: the operation is difficult, slow, and costly; the initial amount of RNA is required to be high; only short fragments of RNA can be detected; serious Bias are induced during the construction of library.

### Contents of the present invention

The present invention provides an RNA library construction method and a specialized kit thereof.

The present invention provides a first method (Method 1) for preparing an RNA library, comprising the following steps:
extracting RNA, and performing fragmentation;
adding a tail to 3' end;
ligating an adaptor to 5' end, and hybridizing with a DNA probe mixture; the DNA probe mixture is composed of several DNA probes that are reverse complementary to an RNA that is expected to be removed;
removing RNA from the hybrid and removing DNA;
performing reverse transcription and PCR amplification to obtain a library solution.

The method 1 preferably comprises the following steps:
(1) extracting RNA and performing fragmentation;
(2) adding a tail to 3' end;
(3) ligating an adaptor to 5' end and hybridizing with a DNA probe mixture; the DNA probe mixture is composed of several DNA probes that are reverse complementary to an RNA that is expected to be removed;
(4) removing RNA from the hybrid and removing DNA;
(5) performing reverse transcription and PCR amplification in sequence to obtain a library solution.

The fragmentation is implemented by heat treatment. The purpose of the fragmentation is to obtain an RNA fragment of below 400 nt. When RIN>8, the heat treatment has parameters of: 94°C, 10 minutes. When 5≤RIN≤8, the heat treatment has parameters of: 90°C, 5 minutes. When 3≤RIN≤5, the heat treatment has parameters of: 80°C, 5 minutes. When RIN<3, the heat treatment has parameters of: 65°C, 5 minutes.

The step of adding a tail to 3' end is implemented by the following manner: modifying an RNA fragment to have a phosphate group at its 5' end and a hydroxyl group at its 3' end, and then adding a tail. The step of "modifying an RNA fragment to have a phosphate group at its 5' end and a hydroxyl group at its 3' end" is implemented by the following method: treating with T4 PNK

The step of adding a tail refers to adding a poly(A) tail or poly(U) tail.

The step of removing RNA from the hybrid and removing DNA is implemented by the following method: performing RNaseH digestion and DNase digestion sequentially.

The step of extracting RNA refers to extracting CfRNA or extracting total RNA.

The sample used for extracting RNA includes but is not limited to plasma, tissue or cell.

The present invention also seeks to protect a method for preparing an RNA library (Method 2), comprising the following steps:
extracting RNA;
adding a tail to 3' end;
ligating an adaptor to 5' end and hybridizing with a DNA probe mixture; the DNA probe mixture is composed of several DNA probes that are reverse complementary to an RNA that is expected to be removed;
removing RNA from the hybrid and removing DNA;
performing reverse transcription and PCR amplification to obtain a library solution.

The method 2 preferably comprises the following steps:
(1) extracting RNA;
(2) adding a tail to 3' end;
(3) ligating an adaptor to 5' end and hybridizing with a DNA probe mixture; the DNA probe mixture is composed of several DNA probes that are reverse complementary to an RNA that is expected to be removed;
(4) removing RNA from the hybrid and removing DNA;
(5) performing reverse transcription and PCR amplification in sequence to obtain a library solution.

The step of adding a tail refers to adding a poly(A) tail or poly(U) tail.

The step of removing RNA from the hybrid and removing DNA is implemented by the following method: performing RNaseH digestion and DNase digestion sequentially.

The step of extracting RNA refers to extracting CfRNA or extracting total RNA.

The sample used for extracting RNA includes but is not limited to plasma, tissue or cell.

The present invention also provides a method for preparing an RNA library (Method 3), comprising the following steps:
extracting RNA and performing fragmentation;
adding a tail to 3' end;
ligating an adaptor to 5' end and enriching a target RNA fragment;
performing reverse transcription and PCR amplification to obtain a library solution.

The method 3 preferably comprises the following steps:
(1) extracting RNA and performing fragmentation
(2) add a tail to 3' end;
(3) ligating an adaptor to 5' end and enriching a target RNA fragment;
(4) performing reverse transcription and PCR amplification in sequence to obtain a library solution.

The fragmentation is implemented by heat treatment. The purpose of the fragmentation is to obtain an RNA fragment of below 400 nt. When RIN>8, the heat treatment has parameters of: 94°C, 10 minutes. When 5≤RIN≤8, the heat treatment has parameters of: 90°C, 5 minutes. When 3≤RIN≤5, the heat treatment has parameters of: 80°C, 5 minutes. When RIN<3, the heat treatment has parameters of: 65°C, 5 minutes.

The step of adding a tail to 3' end is implemented by the following manner: modifying an RNA fragment to have a phosphate group at its 5' end and a hydroxyl group at its 3' end, and then adding a tail. The step of "modifying an RNA fragment to have a phosphate group at its 5' end and a hydroxyl group at its 3' end" is implemented by the following method: treating with T4 PNK

The step of adding a tail refers to adding a poly(A) tail or poly(U) tail.

The step of enriching a target RNA fragment is implemented by a method that includes but is not limited to: chip capture, multiplex PCR, or degenerate PCR.

The step of extracting RNA refers to extracting CfRNA or extracting total RNA.

The sample used for extracting RNA includes but is not limited to plasma, tissue or cell.

The present invention also seeks to protect a method for preparing an RNA library (Method 4), comprising the following steps:
extracting RNA;
adding a tail to 3' end;
ligating an adaptor to 5' end and enriching a target RNA fragment;
performing reverse transcription and PCR amplification to obtain a library solution.

The method 4 preferably comprises the following steps:
(1) extracting RNA;
(2) add a tail to 3' end;
(3) ligating an adaptor to 5' end and enriching a target RNA fragment;
(4) performing reverse transcription and PCR amplification in sequence to obtain a library solution.

The step of adding a tail refers to adding a poly(A) tail or poly(U) tail.

The step of enriching a target RNA fragment is implemented by a method that includes but is not limited to: chip capture, multiplex PCR, or degenerate PCR.

The step of extracting RNA refers to extracting CfRNA or extracting total RNA.

The sample used for extracting RNA includes but is not limited to plasma, tissue or cell.

Any one of the aforementioned hybrids is a hybrid of DNA and RNA.

Any one of the aforementioned RNAs is a human RNA.

The RNA that is expected to be removed as described above is rRNA and Y RNA. Any one of the aforementioned DNA probe mixtures is composed of 204 kinds of probes, as shown successively in SEQ ID NO: 5 to SEQ ID NO: 208 of the sequence listing.

The RNA that is expected to be removed as described above is rRNA. Any one of the aforementioned DNA probe mixtures is composed of 195 kinds of probes, as shown successively in SEQ ID NO: 5 to SEQ ID NO: 199 of the sequence listing.

Any one of the aforementioned adaptors is shown in SEQ ID NO: 1 of the sequence listing.

Any one of the aforementioned reverse transcriptions uses a reverse transcription primer as shown in SEQ ID NO: 2 of the sequence listing.

Any one of the aforementioned PCRs uses two primers, one is shown in SEQ ID NO: 3 of the sequence listing, and the other is shown in SEQ ID NO: 4 of the sequence listing.

The present invention also seeks to protect a kit (Kit 1) for preparing an RNA library, which comprises a component A, a component B, a component C, a component D, a component E, and a component F.

The present invention also seeks to protect a kit (Kit 2) for preparing an RNA library, which comprises a component A, a component G, a component D, a component E, and a component F.

The present invention also seeks to protect a kit (Kit 3) for preparing an RNA library, which comprises a component A, a component H, a component B, a component C, a component D, a component E, and a component F;
The present invention also seeks to protect a kit (Kit 4) for preparing an RNA library, which comprises a component A, a component H, a component G, a component D, a component E, and a component F.

The present invention also seeks to protect a kit (Kit 5) for preparing an RNA library, which comprises a component I, a component B, a component C, a component D, a component E and a component F.

The present invention also seeks to protect a kit (Kit 6) for preparing an RNA library, which comprises a component I, a component G, a component D, a component E, and a component F.

Component A is a reagent or a combination of reagents for adding a tail to the 3' end of an RNA molecule.

Component B is a reagent or a combination of reagents for ligating an adaptor to the 5' end of an RNA molecule.

Component C is a DNA probe mixture. The DNA probe mixture is composed of several DNA probes that are reverse complementary to an RNA that is expected to be removed.

Component D is a reagent or a combination of reagents for removing RNA from the hybrid and removing DNA.

Component E is a reagent or a combination of reagents for reverse transcription.

Component F is a reagent or a combination of reagents for PCR.

Component G is a reagent or a combination of reagents for ligating an adaptor to the 5' end of an RNA molecule and for hybridizing with a DNA probe.

Component H is a reagent or a combination of reagents for modifying an RNA fragment to have a phosphate group at its 5' end and a hydroxyl group at its 3' end.

Component I is a reagent or a combination of reagents for tailing and modifying the 3' end of an RNA molecule.

Any one of the aforementioned kits further comprises a component J; the component J is a reagent or a combination of reagents for extracting RNA.

The component A is polyA Polymerase and ATP. The component A is RNase H Buffer, ATP, RNase Inhibitor and polyA Polymerase.

The component B is an adaptor and T4 RNA Ligase 1.

The component D is RNase H and DNase. The component D is RNase H, Turbo DNase and RNase-free DNase I. The component D is RNase H, Turbo DNase, RNase-free DNase I, CaCl₂ and Tris-Cl buffer.

The component E is a reverse transcription primer and HiScript II RT. The component E is a reverse transcription primer, dNTP, RNase Inhibitor, HiScript II Buffer and HiScript II RT.

The component F is an amplification primer pair. The component F is an amplification primer pair and HiFi PCR Mix.

The component G is an adaptor, a DNA probe mixture and T4 RNA Ligase 1. The component G is an adaptor, a DNA probe mixture, T4 RNA Ligase 1, T4 RNA Ligase Buffer, ATP, PEG8000, RNase Inhibitor, alkaline phosphatase and Tris-Cl buffer.

The component H is T4 PNK

The component I is polyA Polymerase, ATP and T4 PNK. The component I is RNase H Buffer, ATP, RNase Inhibitor, T4 PNK and polyA Polymerase.

The step of adding a tail refers to adding a poly(A) tail or poly(U) tail.

The step of extracting RNA refers to extracting CfRNA or extracting total RNA.

The sample used for extracting RNA includes but is not limited to plasma, tissue or cell.

Any one of the aforementioned hybrids is a hybrid of DNA and RNA.

Any one of the aforementioned RNAs is a human RNA.

The RNA that is expected to be removed as described above is rRNA and Y RNA. Any one of the aforementioned DNA probe mixtures is composed of 204 kinds of probes, as shown successively in SEQ ID NO: 5 to SEQ ID NO: 208 in the sequence listing.

The RNA that is expected to be removed as described above is rRNA. Any one of the aforementioned DNA probe mixtures is composed of 195 kinds of probes, as shown successively in SEQ ID NO: 5 to SEQ ID NO: 199 in the sequence listing.

Any one of the aforementioned adaptors is shown in SEQ ID NO: 1 of the sequence listing.

Any one of the aforementioned reverse transcription primers is shown in SEQ ID NO: 2 of the sequence listing.

For any one of the aforementioned amplification primer pairs, one is shown in SEQ ID NO: 3 of the sequence listing, and the other is shown in SEQ ID NO: 4 of the sequence listing.

The present invention also seeks to protect a use of any one of the aforementioned methods in sequencing. The sequencing is high-throughput sequencing.

The present invention also seeks to protect a use of any one of the aforementioned kits in sequencing. The sequencing is high-throughput sequencing.

The present invention combines polyA tailing and 5'end ligation. The RNA content in the system can be quickly increased by polyA tailing, thereby avoiding subsequent purification losses. It is convenient to remove specific sequences. Through fragmentation and modification (T4 PNK treatment), all types of RNA enter a similar chemical state, and can be constructed into library at the same time.

### Brief Description of the Drawings

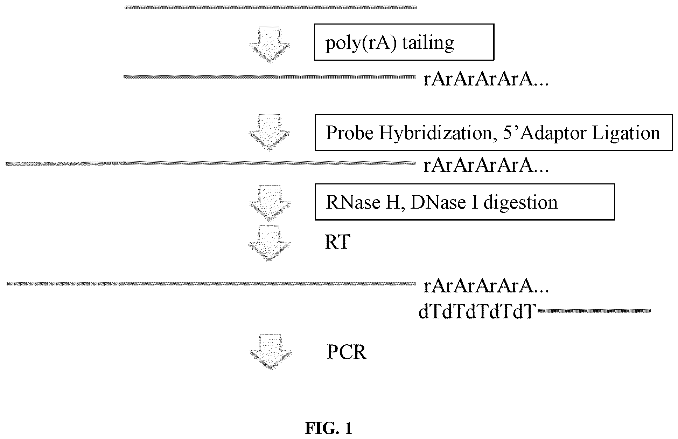

FIG. 1 shows a schematic flowchart of the method provided by the present invention.

### Best Models for Carrying Out the present invention

The following examples facilitate a better understanding of the present invention, but do not limit the present invention. The experimental methods in the following examples are conventional methods unless otherwise specified. The test materials used in the following examples, unless otherwise specified, are all purchased from conventional biochemical reagent stores. The quantitative tests in the following examples are all set to three repeated experiments, and the results are averaged.

10×RNase H Buffer, RNase H: NEB M0297L. RNase Inhibitor: Thermo EO0382. T4 PNK NEB M0201. polyA Polymerase: NEB M0276. 10×T4 RNA Ligase Buffer, T4 RNA Ligase 1: NEB M0204. The alkaline phosphatase used in the examples was FastAP alkaline phosphatase: Thermo EF0651. Turbo DNase: Thermo AM2238. RNase-free DNase I: NEB M0303. RNA Clean Beads: Vazyme N411. DNA Clean Beads: Vazyme N412. HiScript II RT, 5×HiScript II Buffer: Vazyme R201. 2×HiFi PCR Mix: KAPA KK2602. Qubit HS: Thermo Q32854.

Small-fragment RNA refers to an RNA molecule with a size of 18nt to 300nt (including 18nt and 300nt). Common small-fragment RNAs include but are not limited to microRNA, short noncoding RNA (scRNA, tRNA, snRNA, snoRNA, piRNA).

Large-fragment RNA refers to an RNA molecule with a size of larger than 300 nt. Common large-fragment RNAs include but are not limited to mRNA and LncRNA (long noncoding RNA).

In SEQ ID NO: 5 to SEQ ID NO: 208 in the sequence listing, R represents A or G.

### Example 1: Using CfRNA as sample to construct RNA library (containing information of small-fragment RNA)

### I. RNA extraction

CfRNA was extracted from 300µL of plasma (the amount of RNA was about 12ng).

### II. RNA library construction

### 1. Adding polyA.

The CfRNA obtained in step I was taken, added with DEPC H₂O to reach a volume of 14.5µL, then added with 2µL of 10×RNase H Buffer, then placed on ice, then added with the reagents according to Table 1, and then allowed to react at 37°C for 30 minutes, then placed on ice, and then added with 2µL of 40mM EDTA aqueous solution to stop the reaction.

**Table 1**

| Reagent | Volume |
|---|---|
| 10mM ATP | 2 µL |
| 40U/µL RNase Inhibitor | 0.5 µL |
| DEPC water | 0.5 µL |
| 5U/µL poly A Polymerase | 0.5 µL |

### 2. Hybridization with rRNA probe and ligation of adaptor.

After the completion of step 1, the system was added with 1µL of Depletion Probe Set Mix and 0.5µL of 20µM adaptor and allowed to react (reaction conditions: 95°C for 5 minutes, then cooled down to 22°C at a rate of 0.1°C/sec, and then held at 22°C for 5 minutes), then placed on ice, then added with the reagents according to Table 2, and allowed to react for 1 hour at 25°C, then added with 2µL of 1U/µL alkaline phosphatase and 13µL of Tris-Cl buffer (pH7.5, 10mM), and then allowed to react at 37°C for 10 minutes.

adaptor (SEQ ID NO: 1 in the sequence listing): 5'-GAACGACAUGGCUACGAUCCGACUUNNNN-3'.

In the adaptor, N represents A, G, C or U. Both the 5' end and the 3' end of the adaptor have hydroxyl group.

**Table 2**

| Reagent | Volume |
|---|---|
| 10 × T4 RNA Ligase Buffer | 4 µL |
| 10mM ATP | 4 µL |
| 50% PEG8000 | 7 µL |
| 10U/µL T4 RNA Ligase 1 | 1 µL |
| 40U/µL RNase Inhibitor | 0.5 µL |

Depletion Probe Set Mix was a probe mixture solution (the probe mixture was composed of 204 kinds of probes, as shown successively in SEQ ID NO: 5 to SEQ ID NO: 208 of the sequence listing; in the Depletion Probe Set Mix, the concentration of each probe was 0.5µM). Each probe in the primer mixture was reverse complementary to an RNA (rRNA and Y RNA) that was expected to be removed, and annealed to form a DNA:RNA hybrid.

### 3. Removal of DNA and RNA expected to be removed

(1) After the completion of step 2, the system was added with 1µL of 5U/µL RNase H and 4µL of Tris-Cl buffer (pH7.5, 10mM), then allowed to react at 37°C for 30 minutes, then placed on ice, and then added with the reagents according to Table 3, then allowed to react at 37°C for 30 minutes, then placed on ice.

**Table 3**

| Reagent | Volume |
|---|---|
| 30mM CaCl₂ | 3 µL |
| 2U/µL Turbo DNase | 1 µL |
| 2U/µL RNase-free DNase I | 2 µL |
| Tris-Cl buffer (pH7.5, 10mM) | 4 µL |

(2) After the completion of step (1), the system was added with 70µL of RNA Clean Beads (1X) for purification, the purified product was collected with 15µL of DEPC water, and 12.5µL of supernatant was carefully pipetted into a new Nuclease-free centrifuge tube.

### 4. Reverse transcription and PCR

(1) The centrifuge tube of step 3 (2) was taken, added with 1µL of 5µM Ad153R-25dT-VN, allowed to react at 65°C for 5 minutes, then placed on ice for 2 minutes, then added with the reagents according to Table 4, and then subjected to reverse transcription (reverse transcription program: 50°C, 40 minutes), then added with 25µL of 3mM EDTA aqueous solution, mixed well, and allowed to react at 85°C for 15 minutes.

**Table 4**

| Reagent | Volume |
|---|---|
| 5×HiScript II Buffer | 4 µL |
| 10mM dNTP | 1 µL |
| 40U/µL RNase Inhibitor | 0.5 µL |
| 200U/µL Hi Script II RT | 1 µL |

| | |
|---|---|
| Ad153R-25dT-VN (SEQ ID NO: 2 of the sequence listing): 5'-GACCGCTTGGCCTCCGACTTTTTTTTTTTTTTTTTTTTTTTTTVN-3'. | |

In the Ad153R-25dT-VN, V represents A, G or C, and N represents A, G, C or T.
(2) After the completion of step (1), 2µL of product was taken, added with the reagents according to Table 5, and then subjected to PCR amplification (PCR amplification reaction program: 94°C for 3min; 94°C for 20s, 60°C for 20s, 72°C for 30s, 20 cycles; 72°C for 5min; stored at 16°C).

**Table 5**

| | Volume |
|---|---|
| 10µM Pi-Ad153F-FL | 2 µL |
| 10µM Ad153-RT-Barcode-N | 2 µL |
| 2×HiFi PCR Mix | 25 µL |

| | |
|---|---|
| Ad153-RT-Barcode-N (SEQ ID NO: 3 of the sequence listing): 5'-TGTGAGCCAAGGAGTTGNNNNNNNNNNTTGTCTTCCTAAGACCGCTTGGCCTC CGACTT-3'. | |

In the Ad153-RT-Barcode-N, the 10 "N"s marked in the bounding box were sample barcode, and each sample had a unique sample barcode.
Pi-Ad153F-FL (SEQ ID NO: 4 of the sequence listing): 5'-GAACGACATGGCTACGATCCGACTT-3'.
(3) After the completion of step (2), the system was added with 90µL of DNA Clean Beads (1.8X) for purification, and the purified product was collected with 20µL of 1×TE solution, which was the library solution.

### 5. Quality control of library

Quantitation of the library solution was carried out with Qubit HS, and the library concentration should be greater than 2ng/µL.

### III. High-throughput sequencing

The library solutions of multiple samples were mixed and then subjected to high-throughput sequencing.

The instrument used was BGI-SEQ500RS.

The parameters settings were: SE35; 4 pool 1; other settings were BGI-SEQ500RS standard Protocol.

Low-quality Reads were removed; the 4 bases at the 5' end and the "A"s at the 3' end (until the first base that was not A) of the Reads were removed; the Reads that contained at least 15 consecutive "A"s were removed; the Reads that were no longer than 17 bases were removed; rRNA alignment and removal were performed;

MicroRNA and piRNA alignments were performed, and the number of Reads of each gene compared to the number of total Reads was calculated.

### Example 2: Comparison of effects

The plasma samples were 30 human plasma samples.

RNA libraries were constructed according to the method described in Example 1, the average genome alignment rate was 58.72%, and the average transcriptome alignment rate was 39.59%.

Libraries were constructed according to the RNaseH method (http://www.vazyme.com/downloadRepository/11bf871d-c66c-40cc-adbe-317a78af2170.pdf), the average genome alignment rate was 62.44%, and the average transcriptome alignment rate was 26.99% (the alignment rate here was an artifact caused by mitochondrial rRNA, the actual alignment rate was about 3%).

Libraries were constructed according to the Small RNA (http://www.vazyme.com/downloadRepository/32ab9b89-8079-4961-b571-daf964c02a46.pdf), the average genome alignment rate was 4.38%, and the average transcriptome alignment rate was 1.77%.

Libraries were constructed according to the SMARTer (SMARTer® Stranded Total RNA-Seq Kit-Pico Input Mammalian), the average genome alignment rate was 35.10%, and the average transcriptome alignment rate was 37.23%.

### Example 3: Using CfRNA as sample to construct RNA library (containing both large-fragment RNA information and small-fragment RNA information)

### I. RNA extraction

CfRNA was extracted from 300µL of plasma (the amount of RNA was about 12ng).

### II. RNA library construction

### 1. Fragmentation, modification, and adding polyA.

The fragmentation was implemented by heat treatment.

The purpose of the modification was to make the RNA fragment obtained by the fragmentation form a 5'-end phosphate group and a 3'-end hydroxyl group.

The CfRNA obtained in step I was taken, added with DEPC H₂O to reach a volume of 14.5µL, then added with 2µL of 10×RNase HBuffer, subjected to heat treatment (when RIN>8, the heat treatment had parameters of 94°C, 10 minutes; when 5≤RIN≤8, the heat treatment had parameters of 90 °C, 5 minutes; when 3≤RIN≤5, the heat treatment had parameters of 80°C, 5 minutes; when RIN<3, the heat treatment had parameters of 65°C, 5 minutes), then placed on ice, and then added with the reagents according to Table 6, then allowed to react at 37°C for 30 minutes, then placed on ice, and then added with 2 µL of 40 mM EDTA aqueous solution to stop the reaction.

**Table 6**

| Reagent | Volume |
|---|---|
| 10mM ATP | 2 µL |
| 40U/µL RNase Inhibitor | 0.5 µL |
| 10U/µL T4 PNK | 0.5 µL |
| 5U/µL poly A polymerase | 0.5 µL |

Steps 2 to 5 were the same as steps 2 to 5 of step II in Example 1.

### 3. High-throughput sequencing

Same as the step III of Example 1.

### Example 4. Using total RNA as sample to construct RNA library

### I. RNA extraction

Total RNA was extracted from a tissue or cells.

### II. RNA library construction

### 1. Fragmentation, modification, and adding polyA.

The fragmentation was implemented by heat treatment.

The purpose of the modification was to make the RNA fragment obtained by the fragmentation form a 5'-end phosphate group and a 3'-end hydroxyl group.

The total RNA (about 12ng) obtained in step I was taken, added with DEPC H₂O to reach a volume of 14.5µL, then added with 2µL of 10×RNase HBuffer, subjected to heat treatment (when RIN>8, the heat treatment had parameters of 94°C, 10 minutes; when 5≤RIN≤8, the heat treatment had parameters of 90°C, 5 minutes; when 3≤RIN≤5, the heat treatment had parameters of 80°C, 5 minutes; when RIN<3, the heat treatment had parameters of 65°C, 5 minutes), then placed on ice, and then added with the reagents according to Table 6, then allowed to react at 37°C for 30 minutes, then placed on ice, and then added with 2 µL of 40 mM EDTA aqueous solution to stop the reaction.

### 2. Hybridization with rRNA probe and ligation of adaptor.

After the completion of step 1, the system was added with 1µL of Depletion Probe Set Mix and 0.5µL of 20µM adaptor and allowed to react (reaction conditions: 95°C for 5 minutes, then cooled down to 22°C at a rate of 0.1°C/sec, and then held at 22°C for 5 minutes), then placed on ice, then added with the reagents according to Table 2, then allowed to react for 1 hour at 25°C, then added with 2µL of 1U/µL alkaline phosphatase and 13µL of Tris-Cl buffer (pH7.5, 10mM), then allowed to react at 37°C for 10 minutes.

adaptor (SEQ ID NO: 1 in the sequence listing): 5'-GAACGACAUGGCUACGAUCCGACUUNNNN-3' .

In the adaptor, N represents A, G, C or U. Both the 5' end and the 3' end of the adaptor have hydroxyl group.

Depletion Probe Set Mix was a probe mixture solution (the probe mixture was composed of 195 kinds of probes, as shown successively in SEQ ID NO: 5 to SEQ ID NO: 199 of the sequence listing; in the Depletion Probe Set Mix, the concentration of each probe was 0.5µM). Each probe in the primer mixture was reverse complementary to an RNA (rRNA) that was expected to be removed, and annealed to form a DNA:RNA hybrid.

Steps 3 to 5 were the same as steps 3 to 5 of step II of Example 1.

### III. High-throughput sequencing

The library solutions of multiple samples were mixed and then subjected to high-throughput sequencing.

The instrument used was BGI-SEQ500RS.

The parameter settings were: SE50; 4 pool 1; other settings were BGI-SEQ500RS standard Protocol.

Low-quality Reads were removed; the 4 bases at the 5' end and the "A"s at the 3' end (until the first base that was not A) of the Reads were removed; the Reads that contained at least 15 consecutive "A"s were removed; the Reads that were no longer than 17 bases were removed; rRNA alignment and removal were performed;

MicroRNA and piRNA alignments were performed, and the number of Reads of each gene compared to the number of total Reads was calculated.

RNA-RefSeq alignment was performed, alignments of short noncoding RNA, mRNA and Long Noncoding were performed separately, and RPKM was calculated.

### Example 5: Using total RNA as sample to construct RNA library

### I. RNA extraction

Total RNA was extracted from a tissue or cells.

### II. RNA library construction

### 1. Adding polyA.

The total RNA (about 12ng) obtained in step I was taken, added with DEPC H₂O to reach a volume of 14.5µL, then added with 2µL of 10×RNase H Buffer, then placed on ice, then added with the reagents according to Table 1, and allowed to react at 37°C for 30 minutes, then placed on ice, then added with 2µL of 40mM EDTA aqueous solution to stop the reaction.

### 2. Hybridization with rRNA probe and ligation of adaptor.

Same as step 2 of step II in Example 4.
Steps 3 to 5 were the same as steps 3 to 5 of step II in Example 1.

### III. High-throughput sequencing

Same as step III in Example 4.

### Example 6: Comparison of effects

Samples were placental tissue sample and human immortalized leukocyte (by EB virus method) sample.

RNA libraries were constructed according to the method described in Example 4, the genome alignment rate of the placental tissue sample was 66.32%, the microRNA alignment rate was 0.32%, and the mRNA+lncRNA alignment rate was 27.76%.

RNA libraries were constructed according to the method described in Example 5, the genome alignment rate of the placental tissue sample was 79.18%, the microRNA alignment rate was 21.54%, and the mRNA+lncRNA alignment rate was 9.50%.

RNA libraries were constructed according to the method described in Example 4, the genome alignment rate of the human immortalized leukocyte sample was 73.07%%, the microRNA alignment rate was 0.22%, and the mRNA+lncRNA alignment rate was 28.52%.

RNA libraries were constructed according to the method described in Example 5, the genome alignment rate of the human immortalized leukocyte sample was 86.85%, the microRNA alignment rate was 2.41%, and the mRNA+lncRNA alignment rate was 20.40%.

### Industrial application

The present invention has the following advantages: the library construction has high efficiency and high sensitivity; the process is simplified and greatly accelerated; it is very low-cost, with one reaction material less than 30 yuan; the operation is simple; it is easy to customize, and modules can be adjusted to suit different needs; it has high quality of sequencing; it is capable of detecting long-fragment and short-fragment RNA at the same time.

## Claims

**1.** A method for preparing an RNA library, comprising the following steps:
extracting RNA, and performing fragmentation;
adding a tail to 3' end;
ligating an adaptor to 5' end, and hybridizing with a DNA probe mixture; the DNA probe mixture is composed of several DNA probes that are reverse complementary to an RNA that is expected to be removed;
removing RNA from the hybrid and removing DNA;
performing reverse transcription and PCR amplification to obtain a library solution.

**2.** The method according to claim 1, wherein the fragmentation is implemented by heat treatment.

**3.** The method according to claim 1, wherein the step of adding a tail to 3' end is implemented by the following manner: modifying an RNA fragment to have a phosphate group at its 5' end and a hydroxyl group at its 3' end, and then adding a tail.

**4.** The method according to claim 3, wherein: the step of "modifying an RNA fragment to have a phosphate group at its 5' end and a hydroxyl group at its 3' end" is implemented by the following method: treating with T4 PNK

**5.** The method according to any one of claims 1 to 4, wherein the step of removing RNA from the hybrid and removing DNA is implemented by the following method: performing RNaseH digestion and DNase digestion sequentially.

**6.** A method for preparing an RNA library, comprising the following steps:
extracting RNA;
adding a tail to 3' end;
ligating an adaptor to 5' end and hybridizing with a DNA probe mixture; the DNA probe mixture is composed of several DNA probes that are reverse complementary to an RNA that is expected to be removed;
removing RNA from the hybrid and removing DNA;
performing reverse transcription and PCR amplification to obtain a library solution.

**7.** The method according to claim 6, wherein the step of removing RNA from the hybrid and removing DNA is implemented by the following method: performing RNaseH digestion and DNase digestion sequentially.

**8.** A method for preparing an RNA library, comprising the following steps:
extracting RNA and performing fragmentation;
adding a tail to 3' end;
ligating an adaptor to 5' end and enriching a target RNA fragment;
performing reverse transcription and PCR amplification to obtain a library solution.

**9.** A method for preparing an RNA library, comprising the following steps:
extracting RNA;
adding a tail to 3' end;
ligating an adaptor to 5' end and enriching a target RNA fragment;
performing reverse transcription and PCR amplification to obtain a library solution.

**10.** A kit for preparing an RNA library, comprising a component A, a component B, a component C, a component D, a component E, and a component F;
Component A is a reagent or a combination of reagents for adding a tail to the 3' end of an RNA molecule;
Component B is a reagent or a combination of reagents for ligating an adaptor to the 5' end of an RNA molecule;
Component C is a DNA probe mixture; the DNA probe mixture is composed of several DNA probes that are reverse complementary to an RNA that is expected to be removed;
Component D is a reagent or a combination of reagents for removing RNA from the hybrid and removing DNA;
Component E is a reagent or a combination of reagents for reverse transcription;
Component F is a reagent or a combination of reagents for PCR.

**11.** A kit for preparing an RNA library, comprising a component A, a component G, a component D, a component E, and a component F;
Component A is a reagent or a combination of reagents for adding a tail to the 3' end of an RNA molecule;
Component G is a reagent or a combination of reagents for ligating an adaptor to the 5' end of an RNA molecule and for hybridizing with a DNA probe;
Component D is a reagent or a combination of reagents for removing RNA from the hybrid and removing DNA;
Component E is a reagent or a combination of reagents for reverse transcription;
Component F is a reagent or a combination of reagents for PCR.

**12.** A kit for preparing an RNA library, comprising a component A, a component H, a component B, a component C, a component D, a component E, and a component F;
Component A is a reagent or a combination of reagents for adding a tail to the 3' end of an RNA molecule;
Component H is a reagent or a combination of reagents for modifying an RNA fragment to have a phosphate group at its 5' end and a hydroxyl group at its 3' end;
Component B is a reagent or a combination of reagents for ligating an adaptor to the 5' end of an RNA molecule;
Component C is a DNA probe mixture; the DNA probe mixture is composed of several DNA probes that are reverse complementary to an RNA that is expected to be removed;
Component D is a reagent or a combination of reagents for removing RNA from the hybrid and removing DNA;
Component E is a reagent or a combination of reagents for reverse transcription;
Component F is a reagent or a combination of reagents for PCR.

**13.** A kit for preparing an RNA library, comprising a component A, a component H, a component G, a component D, a component E, and a component F;
Component A is a reagent or a combination of reagents for adding a tail to the 3' end of an RNA molecule;
Component H is a reagent or a combination of reagents for modifying an RNA fragment to have a phosphate group at its 5' end and a hydroxyl group at its 3' end;
Component G is a reagent or a combination of reagents for ligating an adaptor to the 5' end of an RNA molecule and for hybridizing with a DNA probe;
Component D is a reagent or a combination of reagents for removing RNA from the hybrid and removing DNA;
Component E is a reagent or a combination of reagents for reverse transcription;
Component F is a reagent or a combination of reagents for PCR.

**14.** A kit for preparing an RNA library, comprising a component I, a component B, a component C, a component D, a component E, and a component F;
Component I is a reagent or a combination of reagents for tailing and modifying the 3' end of an RNA molecule;
Component B is a reagent or a combination of reagents for ligating an adaptor to the 5' end of an RNA molecule;
Component C is a DNA probe mixture; the DNA probe mixture is composed of several DNA probes that are reverse complementary to an RNA that is expected to be removed;
Component D is a reagent or a combination of reagents for removing RNA from the hybrid and removing DNA;
Component E is a reagent or a combination of reagents for reverse transcription;
Component F is a reagent or a combination of reagents for PCR.

**15.** A kit for preparing an RNA library, comprising a component I, a component G, a component D, a component E, and a component F;
Component I is a reagent or a combination of reagents for tailing and modifying the 3' end of an RNA molecule;
Component G is a reagent or a combination of reagents for ligating an adaptor to the 5' end of an RNA molecule and for hybridizing with a DNA probe;
Component D is a reagent or a combination of reagents for removing RNA from the hybrid and removing DNA;
Component E is a reagent or a combination of reagents for reverse transcription;
Component F is a reagent or a combination of reagents for PCR.

**17.** Use of the method according to any one of claims 1 to 9 in sequencing.

**18.** Use of the kit according to any one of claims 10 to 16 in sequencing.
